# EUROPÄISCHE PATENTANMELDUNG

(11) **EP 1 287 814 A1**
(43) Veröffentlichungstag der Anmeldung: **05.03.2003**
(21) Anmeldenummer: 01121130.7
(22) Anmeldetag: 04.09.2001
(51) Int. Cl.: A61K 7/40, A61K 7/48

(54) **Wirkstoffkombination mit Rhodanid (Thiocyanat) als Hauptwirkstoff**

(71) Anmelder: Kramer, Axel, Prof.Dr.med.habil., 17489 Greifswald (DE)
(72) Erfinder: Kramer, Axel, Prof.Dr.med.habil., 17489 Greifswald (DE)
(74) Vertreter: Jönsson, Hans-Peter, Dr.Dipl.-Chem.

(57) **Zusammenfassung**

Die Erfindung betrifft äußerlich anzuwendende Mittel zur Förderung der Abwehrleistung der Haut gegenüber chemischen und physikalischen Irritationen, zum Beispiel bei beruflichen Belastungen, Strapazierung der Haut im Freizeitbereich (Baden, Duschen, Sonneneinwirkung, Haushaltschemikalien, Baustoffe u.a.) und in der Arbeitswelt enthaltend Kombinationen von Rhodanid mit Harnstoff und/oder Harnstoffderivat.

## Beschreibung

Die Erfindung betrifft äußerlich anzuwendende Mittel zur Förderung der Abwehrleistung der Haut gegenüber chemischen und physikalischen Irritationen, zum Beispiel bei beruflichen Belastungen, Strapazierung der Haut im Freizeitbereich (Baden, Duschen, Sonneneinwirkung, Haushaltschemikalien, Baustoffe u.a.) und in der Arbeitswelt enthaltend Kombinationen von Rhodanid mit Harnstoff und/oder dessen Derivate.

Zum Hautschutz wird seit Jahrzehnten eine Vielzahl von Hautschutzpräparaten eingesetzt, ohne dass ihre Wirksamkeit experimentell belegt ist. Üblicherweise wird der Hautschutz dabei dadurch erreicht, dass der Schutz durch eine Expositionsbarriere gegebenenfalls in Verbindung mit einer Erhöhung der Hautfeuchtigkeit, nicht aber durch eine Förderung der Barriere-Funktion und der Abwehrleistung der Haut gegenüber Irritationen auf biochemischem Weg erreicht wird (Elsner P., Wigger-Alberti W. (1997) Der Hautschutz in der Prävention der Berufsdermatosen, Dt. Ärzteblatt 94; 17-1489-A-1492).

Für den Wirkstoff Rhodanid (Thiocyanat) sind als biologische Wirkungen am Hautorgan eine Reihe von Anwendungen experimentell gesichert. Kramer A.: Prüfsystem zur Erfassung der Verträglichkeit antimikrobiell wirksamer Stoffe und Zubereitungen zu episomatischen Applikation durch in-vitro und tierexperimentielle Tests (Episomatiktest) und toxikohygienische Bewertung als Bestandteil krankenhaushygienischer Aufgabenstellungen; Diss. B. Med. Fak. Univ. Greifswald (1985) beschreibt eine beschleunigte Abheilung von Hautwunden. In der DE 41 34 888 A wird die beschleunigte Abheilung von UV induziertem Erythem (Sonnenbrand) beschrieben.

A. Kramer, W. Weuffen, S. Minnich, S. Koch, M. Minnich, H. Below, B. Thürkow und H. Meffert (1990), Förderung der Haarentwicklung durch Thiocyanat beim Meerschweinchen; Dertamto. Mschr. 176: 417-420 beschreiben eine Wachstumsförderung von Hautanhangsgebilden (Haar).

Für den Wirkstoff Harnstoff sind ebenfalls eine Reihe von folgenden Wirkungen bekannt (Wohlrab W. (1989), Bedeutung von Harnstoff in der externen Therapie, Hautarzt 40, Suppl. 9: 35-41), wobei der Anwendungsbereich für die nachfolgend genannten Harnstoffwirkungen jeweils 2 Gew.-% nicht unterschreitet:
Hydratisierung des Stratum corneum, keratolytische Eigenschaften, Beschleunigung der Penetration, Hemmung der epidermalen Proliferation (Basalzellen), geringe antimikrobielle Wirksamkeit, antipruriginöser Effekt, proteo- und mukolytische Aktivität und puffernde Wirkung (Regulation des Hydrolipidmantels).

An therapeutisch nutzbaren Effekten ergeben sich aus diesen Eigenschaften des Harnstoffs (Raab W. (1989) Biochemie, Pharmakologie und Toxikologie von Harnstoff; Hautarzt 40 Suppl.9:23-26):
Hydratisierung der Hornschicht, Keratolyse erkrankter Nägel, Schuppenlösung, Reduzierung überschießender Zellteilungen, Juckreizstillung, Einsparung von Konservierungsmitteln durch antimikrobiellen Effekt und Verbesserung der Penetration eingearbeiteter Pharmaka.

In der modernen Dermatotherapie wird Harnstoff als Monotherapeutikum oder als Zusatzstoff in der Abheilungsphase von Dermatosen erfolgreich angewandt. Als Monotherapeutikum wird Harnstoff bei Ekzemen trockener Haut, bei schuppenden Läsionen, bei Hyperkeratosen und bei vielen anderen chronischen Dermatosen eingesetzt (Swanbeck G. (1989), Harnstoff als Monotherapeutikum bei trockener Haut; Hautarzt 40 Suppl. 9: 42-43)., Harnstoff wird in dermatologischen Externa häufig mit Glukokortikoiden (Drosner M. (1989), Harnstoff in Kombination mit Kortikosteroiden zur Therapie von Ekzemen; Hautarzt 40, Suppl. 9: 47-50), mit Dithranol (Przybilla B., Kaudewitz P., Biber K. (1989), Harnstoff in Kombination mit Dithranol zur Therapie der Psorlasis vulgaris; Hautarzt 40, Suppl.9: 54-57), mit Tretinoin (Müller K.H., Pflugshaupt Ch. (1989); Harnstoff in der Dermatologie; Hautarzt 40, Suppl. 9: 16) und mit Salicylsäure (Gabard B., Bieli E. (1989); Salicylsäure und Harnstoff-mögliche Beeinflussung der keratolytischen Wirkung von Salicylsäure durch Harnstoff; Hautarzt 40, Suppl. 9: 71-73) kombiniert. Bei Neurodermitis, Psoriasis vulgaris und Verhornungsstörungen (Raab loc. cit) wird die regelmäßige Anwendung harnstoffhaltiger Externa zur Rehabilitation der Haut und zur Prophylaxe von Hautläsionen empfohlen, ebenso beim trockenfettarmen Hautzustand. Harnstoff ist, wie aufgrund seines physiologischen Vorkommens im Organismus (Hibber J.M., Jackson A.A. (1991); Variation in measures of urea kinetics over four years in a single adult; Europ. J. clin. Nutr. 45: 347-351) nicht anders zu erwarten, epidermal gut verträglich. Es sind keine sensibilisierende Potenz oder andere Nebenwirkungen des Harnstoffs nachgewiesen (Ashton H., Frenk E., Stevenson C.J. (1971); Urea as a topical agent; Brit. J. Derm. 84: 194-196; Wozniak K.D. (1975); Hauttestungen verschiedener Harnstofftypen; Derm. Mschr. 161: 687).

In US-A-3,384,548 werden Harnstoff (2 bis 7,5 Gew.-%) und Thiocyanationen (4 Gew.-%) zur Depilation von Haar als Quellmittel eingesetzt.

In der DE 43 41 001 A1 wird ein Harnstoffgehalt von 0,1 bis 10 Gew.-% zur Feuchtigkeitsbindung genannt.

In der EP 0 999 819 A ist für die Kombination von Rodanid-Ionen mit Harnstoff im Mischungsverhältnis von 1 : 10 bis 1 : 50 eine Förderung der Abwehrleistung gesunder bzw. beanspruchter Haut durch vorausgehende Anwendung dieser Wirkstoffkombinationen im ModellVersuch an der Chorioallantoismembran (HET-CAM), einem anerkannten Voraussagetest zur Erfassung der Irritationspotenz für Haus und Schleimhaut nachgewiesen.

Der vorliegenden Erfindung liegt die Aufgabe zugrunde, neue noch wirksamere Mittel zur Prophylaxe von Hautschäden durch eine physiologische Hautschutzwirkung auf biochemischem Wege zur Verfügung zu stellen, wobei zugleich Heil- und Reparaturvorgänge stimuliert werden, so dass ein synergistisch verstärkter revitalisierender Hautschutz erreicht wird.

In Untersuchungen am Probanden konnte mit Hilfe der vorliegenden Erfindung der Nachweis erbracht werden, dass durch die Kombination von ionisch gebundenen und/oder freien Thiocyanationen mit Harnstoff neben an sich bekannten Träger- und Hilfsstoffen in einem Mischungsverhältnis von Rhodanid zu Harnstoff mit 10 bis 250 Gew.-Teilen Rhodanid auf 1 Gew.-Teil Harnstoff überraschenderweise eine gegenüber der EP 0 999 819 A verbesserte Wirkung erzielt wird.

Wenn im Sinne der vorliegenden Erfindung die Rede von "Harnstoff" ist, so schließt dieser Begriff auch an sich bekannte Harnstoffderivate, wie beispielsweise Monoacetylharnstoff, Carbamid, Allantoin und deren Gemische mit ein.

Ferner konnte im Explantationstest am Peritonealexplantaten neonataler Ratten nachgewiesen werden (Beschreibung des Testmodells in Kramer A., Adrian V., Rudolph P., Lippert H., Wurster S.: Explantationstest mit Haut und Peritoneum der neonatalen Ratte als Voraussagetest zur Verträglichkeit lokaler Antiinfektiva für Wunden und Körperhöhlen. Chirurg 69 (1998):840-5), dass die zytotoxische Wirkung von Antiseptika auch durch die Kombination von Rhodanid mit Allantoin in größerem Maße reduziert wird als durch den isolierten Einsatz beider Komponenten, wobei Rhodanid allein signifikant wirksamer als Allantoin ist. Daraus lässt sich ableiten, dass die biologische Hauptwirkung zur Förderung der Abwehrleistung von Rhodanid ausgeht und diese durch synergistische Kombinationspartner wie Harnstoff und Allantoin verstärkt wird. Auch die hierbei gefundene antitoxische Wirkung von Allantoin allein war bisher nicht bekannt.

Sowohl bei Thiocyanat als auch bei Harnstoff handelt es sich um physiologische Substrate, die im menschlichen Organismus in verschiedenen Kompartimenten vorkommen, die erfindungsgemäß in der Wirkstoffkombination in einem physiologischen Konzentrationsbereich eingesetzt werden.

Das überraschende Moment der Wirkstoffkombination besteht darin, dass sie im Unterschied zu bekannten Antiphlogistika, die nur erkrankte Gewebe beeinflussen können, bereits bei einer der Irritation vorausgehenden Anwendung diese signifikant in ihrer Ausprägung unterdrücken. Ein derartiges Wirkungsprinzip war bisher nicht bekannt.

Gegenstand der Erfindung in einer ersten Ausführungsform sind äußerlich anzuwendende Mittel zur Förderung der Abwehrleistung der Haut gegenüber chemischen und physikalischen Irritationen, enthaltend ionisch gebundene und/oder freie Thiocyanationen und Harnstoff neben an sich bekannten Hilfs- und Trägerstoffen, dadurch gekennzeichnet, dass das Gewichtsverhältnis von Thiocyanationen zu Harnstoff und/oder Harnstoffderivaten im Bereich von 10 : 1 bis 250 : 1 umfasst.

Die Derivate von Harnstoff umfassen beispielsweise Allantoin.

Besonders bevorzugt im Sinne der vorliegenden Erfindung liegen die Thiocyanationen in Form von Alkalimetallsalzen einschließlich der Ammoniumsalze und ihrer Derivate vor. Hierbei handelt es sich somit um dissoziationsfähige Thiocyanate im Gegensatz zu organischen, kovalent gebundenen Thiocyanaten. Besonders bevorzugt im Sinne der vorliegenden Erfindung sind die Alkalimetallsalze der Thiocyanate, ausgewählt aus Natrium-, Kalium- und/oder Ammoniumthiocyanat.

Im Sinne der vorliegenden Erfindung enthalten die Mittel besonders bevorzugt 10 bis 250 g/kg Thiocyanationen und 0,04 bis 25 g/kg, insbesondere 0,1 bis 10 g/kg Harnstoff und/oder Harnstoffderivat (jeweils bezogen auf das Mittel). Neben den Wirkstoffen Thiocyanat und Harnstoff enthalten die erfindungsgemäßen Mittel übliche Hilfs- und Trägerstoffe beispielsweise für Salben, Cremes, Emulsionen oder Lotionen auf Basis von W/O sowie O/W-Grundlagen oder wässrige Lösungen.

Diese sind beispielsweise aus H.P. Fiedler, Lexikon der Hilfsstoffe für Pharmazie, Kosmetik und angewandte Gebiete 4. Auflage (1996) bekannt, sollen erfindungsgemäß das Verhalten von auf die Haut aufgebrachten Wirkstoffen nicht ändern, wenigstens nicht negativ beeinflussen. Gleiches gilt für die Trägerstoffe.

Die erfindungsgemäße Kombination von Thiocyanat mit Harnstoff führte auf der Grundlage synergistischer Kombinationseffekte zu der überraschenden Hautschutzwirkung der Substanzkombination gegenüber Noxen beziehungsweise den eingangs genannten Belastungen, wobei die bekannte Wirkung von Thiocyanat überraschenderweise durch die Kombination mit Harnstoff verbessert wurde. Mit Hilfe der vorliegenden Erfindung wurde die Möglichkeit eines umfassenden Wirkungsprinzips eines revitalisierenden Hautschutzes verwirklicht.

Eine besondere Ausführungsform der Erfindung besteht in der Verwendung der oben definierten Mittel zur Förderung der Abwehrleistung der Haut gegenüber chemischen und physikalischen Irritationen, insbesondere bei beruflichen Belastungen, Strapazierungen der Haut im Freizeitbereich und in der Arbeitsumwelt. So dienen die erfindungsgemäßen Wirkstoffkombinationen insbesondere dem präventiven Schutz vor aggressiven Medien im Berufsalltag ebenso wie beim Umgang mit Schutzmedien gegen äußere Einflüsse auf die Haut.

### Ausführungsbeispiele:

### Beispiel 1:

Zum Nachweis der antientzündlichen Wirksamkeit des Prüfpräparates, bestehend aus einer handelsüblichen kosmetischen Basiscreme mit Zusatz von Rhodanid (2,5 % NaSCN) und Carbamid (1% Harnstoff) wurden im Vergleich dieselbe kosmetische Basiscreme mit Bisabolol, die kosmetische Basiscreme ohne Wirkstoffzusatz als Placebo, eine 1 %ige Hydrocortisoncreme einer arzneilichen Zubereitung geprüft und die Testfelder zusätzlich mit einem unbehandelten Kontrollfeld sowie einem Leerfeld verglichen.

Mit Hilfe des sogenannten UV-Modells sollte mit den vorgenannten Rezepturen die antiphlogistische Wirksamkeit der vorgenannten Rezepturen überprüft werden. Hierbei erfolgte eine jeweils 1-wöchige Vorbehandlung mit den Prüfpräparaten auf vorgekennzeichneten Testfeldern an den Unterarmen von 10 Probanden mit 1 bis 2 x täglicher Applikation.

Ein Testfeld blieb unbehandelt und diente im Verlauf als Kontrolle, darüber hinaus wurde ein weiteres Testfeld (Leerfeld) im Verlauf der Studie weder behandelt noch bestrahlt.

Nach 1-wöchiger Vorbehandlung wurde auf den Testfeldern mittels eines Punktstrahlers jeweils ein UV-Erythem erzeugt. Die individuelle Erythem-Empfindlichkeit wurde zuvor für jeden der teilnehmenden Probanden bestimmt.

Zur Dokumentation des Erythem-Verlaufs wurden Messungen mit dem Laser-Doppler-Flowmeter zur Bestimmung der kapillaren Hautdurchblutung durchgeführt. Die Messungen fanden an allen Testfeldern zum Zeitpunkt vor Setzen der Erytheme, nach 6 Stunden, 24 Stunden und 48 Stunden statt.

Tabelle 1 zeigt den Anstieg der Durchblutungsänderungen über den Studienverlauf von 48 Stunden.

Es wird deutlich, dass durch die kosmetische Basiscreme mit Rhodanid/Carbamid eine gute antiphlogistische Wirkung erzielt werden konnte, die in der Nähe des Arzneimittels - 1 % Hydrocortisoncreme - lagen (kein signifikanter Unterschied). Nach 48 Stunden war der Ausgangspunkt bereits wieder erreicht.

Im Unterschied dazu war die Basiscreme ohne Wirkstoffzusatz signifikant geringer wirksam. Durch den Zusatz von Bisabolol wurde die antientzündliche Wirksamkeit zwar verbessert, lag aber immer noch signifikant unter der Wirksamkeit der erfindungsgemäßen Kombination.

Am Merkmal der Hautrötung ergab sich eine gleichartige Abstufung in der Wirksamkeit der Prüfzubereitungen.

**Tabelle 1:**

| | Rhodanid/Carbamid* | Bisabolol | Placebo | Hydrocortison | Kontrolle | Leerfeld |
|---|---|---|---|---|---|---|
| Proband | | | | | | |

| vorher | | | | | | |
|---|---|---|---|---|---|---|
| Mittelwert | 0,92 | 0,97 | 0,91 | 0,95 | 0,89 | 0,92 |

| nach 6 h | | | | | | |
|---|---|---|---|---|---|---|
| Mittelwert | 1,54 | 1,98 | 2,37 | 1,30 | 2,42 | 0,85 |

| nach 24 h | | | | | | |
|---|---|---|---|---|---|---|
| Mittelwert | 1,81 | 2,23 | 2,54 | 1,65 | 3,02 | 0,94 |

| nach 48 h | | | | | | |
|---|---|---|---|---|---|---|
| Mittelwert | 1,05 | 1,23 | 1,42 | 1,04 | 1,57 | 0,92 |

| | | | | | | |
|---|---|---|---|---|---|---|
| *erfindungsgemäß | | | | | | |

### Beispiel 2:

Zur Erfassung weiterer Wirkungen auf das Hautorgan, die für die Förderung der Abwehrleistung der Haut gegenüber Noxen von Bedeutung sind, wurde die erfindungsgemäße Creme (s. Beispiel 2) mit der zugrundeliegenden Basiscreme 3 Wochen lang 1 bis 2 mal pro Tag verwendet und feuchtigkeitsanreichernde und hautglättende Wirkung sowie Schuppung verglichen. Die Vergleichsmessung wurde mit einem Corneometer (Courage & Khazaka GmbH, Köln) ermittelt.

Das Profil der Hautoberfläche wurde mit der SELS-Messeinreichung (Surface Evoluation of Living Skin, Courage & Khazaka GmbH, Köln mit einer im Messkopf eingebauten CCD-Kamera aufgenommen und mit Hilfe der Software Skinvisiometer (Courage & Khazaka GmbH, Köln) als Graustufen-Bitmap-Datei übertragen. Die Schuppigkeit wurde mit demselben Messsystem ermittelt. Sowohl bezüglich der Feuchtigkeitsanreicherung, die eine wichtige barrierestabilisierende Wirkung der Haut bewirkt und gleichzeitig die Angriffsmöglichkeit für Noxen reduziert, als auch bezüglich der hautglättenden Wirkung und der Schuppigkeit, die in gleicher Weise zur physiologischen Stabilisierung der Haut beitragen, war die erfindungsgemäße Zubereitung der Basiscreme (Placebo) signifikant (p < 0,01) überlegen (Tabelle 2). Bei den unbehandelten Kontrollen gab es erwartungsgemäß keine signifikanten Veränderungen.

**Tabelle 2:**

| | Mittelwerte | | |
|---|---|---|---|
| Parameter | Rhodanid/Carbamid* | Placebo | Kontrolle |
| Hautfeuchtigkeit (Skalenteile) | | | |
| Ausgangswert (A) | 52,9 | 53,4 | 53 |
| nach 3 Wochen (B) | 67,3 | 64,5 | 56 |

| Hautglättung (SEᵣ) | | | |
|---|---|---|---|
| Ausgangswert (A) | 7,9 | 7,4 | 5,5 |
| nach 3 Wochen (B) | 6,3 | 7,5 | 4,9 |

| Schuppigkeit (Se_{sc}) | | | |
|---|---|---|---|
| Ausgangswert (A) | 16,7 | 12,4 | 11,3 |
| nach 3 Wochen (B) | 3,8 | 5,4 | 9,5 |

| | | | |
|---|---|---|---|
| *erfindungsgemäß | | | |

### Beispiel 3:

Der Wirkungsnachweis der Förderung der Abwehrleistung der Haut gegenüber Noxen wurde im Explantationstest geführt. In diesem Testmodel wurden Peritonealexplantate von 1 mm³ Größe aus Peritoneum neonataler Ratten hergestellt, diese mit den Prüfsubstanzen in einem Uhrglass-Schälchen exponiert, mit Zellzuchtmedium anhaftende Prüfsubstanzreste gründlich abgespült und danach in 24-well-Kulturschalen für 10 Tage kultiviert. Im Vergleich zur Kontrolle mit Ringerlösung (100 %) wurden die Zellauswachszonen um jedes Explantat (in mm²) stereomikroskopisch bestimmt und durch die am 2. Tag der Kultivierung ermittelte Größe des Explantats dividiert, um so die relative Größe der Auswachszonen zu erhalten.

Die relativen Auswachsflächen wurden prozentual auf den Medianwert der relativen Auswachsflächen der als Kontrolle eingesetzten Ringer-Lösung (= 100 %) bezogen und als Wachstumsrate (in %) ausgedrückt, wodurch eine Vergleichbarkeit der Wachstumsraten der verschiedenen Prüfansätze gewährleistet ist (Kramer et al., 1998).

Im Ergebnis entfaltete Rhodanid allein eine signifikant höhere Schutzwirkung bei gleichzeitiger Einwirkung von 70 % Ethanol beziehungsweise 0,05 % Chlorhexidin als Allantoin im Vergleich zur Kontrolle. Überraschend war die synergistische Wirkungsverstärkung bei Anwendung beider Wirkstoffe in Kombination (Tabelle 3). Damit besitzt diese Wirkstoffkombination eine hohe antitoxische Wirksamkeit, die kosmetisch vielfältige Anwendungsbereiche eröffnet.

**Tabelle 3:**

| Antitoxische Hautschutzwirkung im Prüfmodell des Explantationstests mit 70 % Ethanol beziehungsweise 0,05 % Chlorhexidin als Noxe bei 1-minütiger Einwirkungszeit. | | |
|---|---|---|
| Wirkstoff bzw. Prüfgemisch | Zahl der Explantate | Wachstumsrate (% der Kontrolle) Median |
| Ethanol 70 % (E) | 45 | 23 |
| E + 0,5 % Allantoin (A) | 35 | 26¹ |
| E + 405 mg NaSCN/L (SCN)* | 35 | 27² |
| E + A + SCN* | 40 | 32³ |
| Chlorhexidin 0,05 % (CHX) | 46 | 17,8 |
| CHX + A | 47 | 19,5¹ |
| CHX + SCN | 48 | 23,0² |
| CHX + A + SCN | 46 | 29,0³ |

| | | |
|---|---|---|
| *erfindungsgemäß Signifikanter Unterschied zur Einwirkung der isolierten Noxen Ethanol (70 %) bzw. Chlorhexidin (0,05%). | | |
| [1 = p < 0,05] | | |
| [2 = p < 0,01] | | |
| [3 = p < 0,001] | | |

## Patentansprüche

1. Äußerlich anzuwendendes Mittel zur Förderung der Abwehrleistung der Haut gegenüber chemischen und physikalischen Irritationen, enthaltend ionisch gebundene und/oder freie Thiocyanationen und Harnstoff neben an sich bekannten Hilfs- und Trägerstoffen, **dadurch gekennzeichnet, dass** das Gewichtsverhältnis von Thiocyanationen zu Harnstoff und/oder Harnstoffderivaten im Bereich von 10 : 1 bis 250 : 1 umfasst.

2. Mittel nach Anspruch 1, **dadurch gekennzeichnet, dass** sie Allantoin als Harnstoff umfassen.

3. Mittel nach Anspruch 1 oder 2, **dadurch gekennzeichnet, dass** die Thiocyanationen in Form von Alkalimetallsalzen, einschließlich Ammoniumsalzen und ihrer Derivate vorliegen.

4. Mittel nach Anspruch 3, **dadurch gekennzeichnet, dass** die Alkalimetallsalze der Thiocyanate ausgewählt sind aus Natrium-, Kalium- und/oder Ammoniumthiocyanat.

5. Mittel nach einem der Ansprüche 1 bis 4, **dadurch gekennzeichnet, dass** sie 10 bis 250 g/kg Thiocyanationen, bezogen auf das Mittel enthalten.

6. Mittel nach einem der Ansprüche 1 bis 5, **dadurch gekennzeichnet, dass** sie 0,04 bis 25 g/kg Harnstoff und/oder Harnstoffderivat, bezogen auf das Mittel enthalten.

7. Mittel nach einem der Ansprüche 1 bis 6, **dadurch gekennzeichnet, dass** sie 0,1 bis 10 g/kg Harnstoff und/oder Harnstoffderivat, bezogen auf das Mittel enthalten.

8. Mittel nach einem oder mehreren der Ansprüche 1 bis 7, umfassend Salben, Cremes, Emulsionen, Lotionen und/oder alkoholische wässrige Lösungen.

9. Verwendung von ionisch gebundenen und/oder freien Thiocyanationen und Harnstoff neben an sich bekannten Hilfs- und Trägerstoffen mit einem Gewichtsverhältnis von Thiocyanat zu Harnstoff und/oder Harnstoffderivat im Bereich von 10 : 1 bis 250 : 1 zur Herstellung eines Mittels zur Förderung der Abwehrleistung der Haut gegenüber chemischen und physikalischen Irritationen, insbesondere bei beruflichen Belastungen, Strapazierungen der Haut im Freizeitbereich und in der Arbeitsumwelt.
